(19) Europäisches Patentamt
European Patent Office
Office européen des brevets

(11) Veröffentlichungsnummer: 0 358 177
A1

(12) # EUROPÄISCHE PATENTANMELDUNG

(21) Anmeldenummer: 89116418.8

(22) Anmeldetag: 06.09.89

(51) Int. Cl.5: A61K 31/71 , //(A61K31/71, 31:44)

(30) Priorität: 08.09.88 DE 3830509

(43) Veröffentlichungstag der Anmeldung: 14.03.90 Patentblatt 90/11

(84) Benannte Vertragsstaaten: BE CH DE FR GB IT LI NL

(71) Anmelder: HOECHST AKTIENGESELLSCHAFT
Postfach 80 03 20
D-6230 Frankfurt am Main 80(DE)

(72) Erfinder: Seibert, Gerhard, Prof. Dr.
Gläserweg 21
D-6100 Darmstadt(DE)
Erfinder: Raether, Wolfgang, Dr.
Falkensteinstrasse 6
D-6072 Dreieich(DE)
Erfinder: Winkler, Irvin, Dr.
In den Eichen 40
D-6237 Liederbach(DE)
Erfinder: Hammann, Peter, Dr.
Mörikestrasse 6
D-6233 Kelkheim(Taunus)(DE)
Erfinder: Wink, Joachim, Dr.
Bieberer Strasse 133
D-6050 Offenbach am Main(DE)

(54) **Arzneimittel mit synergistischer antimykotischer und antiviraler Wirksamkeit.**

(57) Die Erfindung betrifft Arzneimittel mit synergistischer antimykotischer und antiviraler Wirksamkeit, die mindestens eine Verbindung der Formel I

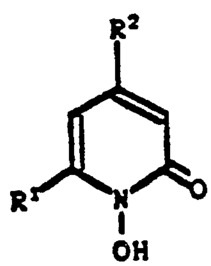

(I)

und mindestens eine Verbindung ausgewählt aus den Nigericinderivaten der Formeln II, III, IV und V,

EP 0 358 177 A1

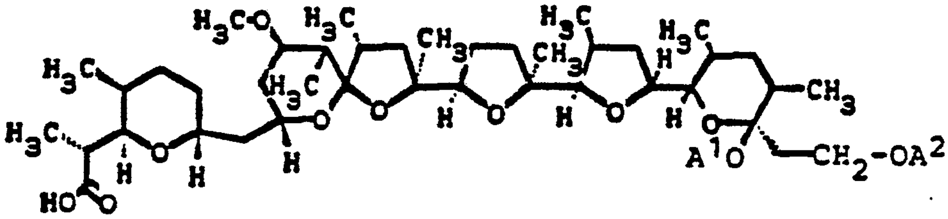

(II)

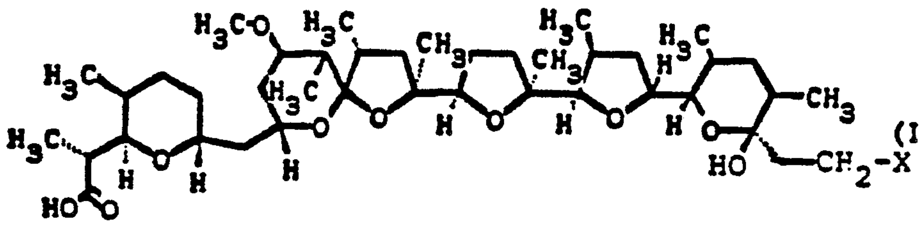

(III)

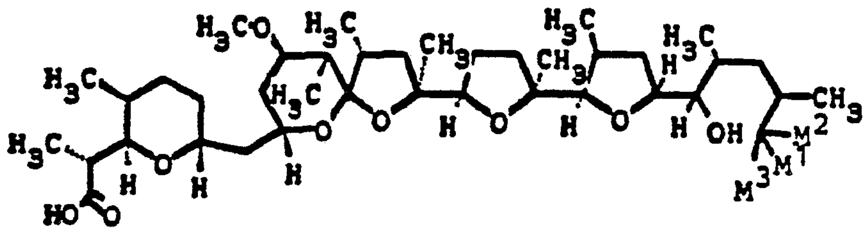

(IV)

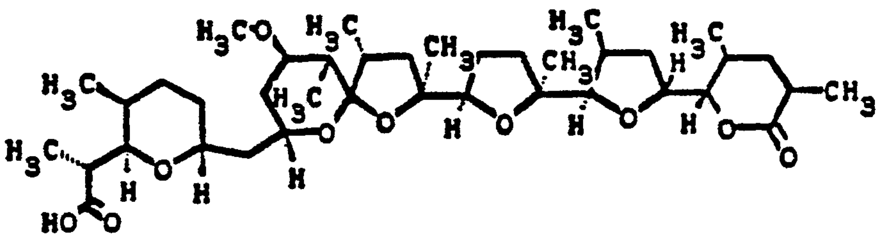

(V)

wobei die Substituenten $R^1$, $R^2$, $A^1$, $A^2$, $M^1$, $M^2$ und $M^3$ die angegebenen Bedeutungen haben, enthalten.

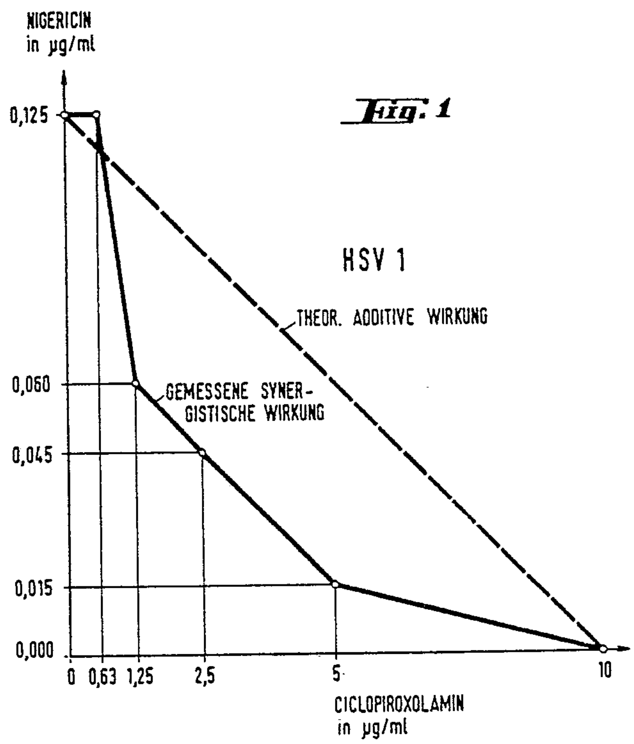

# Arzneimittel mit synergistischer antimykotischer und antiviraler Wirksamkeit

Es ist bekannt, daß Antimykotika aus der Klasser der Ciclopiroxderivate, z. B. das Ethanolamin des 6-Cyclohexyl-1-Hydroxy-4-methyl-2(1H)-Pyridons (Ciclopiroxolamin, ®Batrafen), hervorragend zur Therapie von Pilzinfektionen mit topischer Applikation geeignet sind und außerdem eine zusätzliche antibakterielle Aktivität besitzen (Ciclopiroxolamin, Arzneimittel Forschung, 31, S. 1309-1386). Andererseits ist aber auch bekannt, daß die Aktivität dieser Verbindungen gegenüber manchen Erregern aus der Gruppe der Pilze, der Bakterien und Viren für die Therapie nicht ausreichend sein kann.

Einige Verbindungen aus der Gruppe der Polyether wie Nigericin zeigen eine antibakterielle und antivirale Aktivität. Solche Nigericinderivate wurden bereits vorgeschlagen in den deutschen Patentanmeldungen P 3800598.0, P 3811016.4 und P 3820179.8.

Es wurde nun überraschend gefunden, daß Nigericin und/oder seine Derivate und/oder Salze in Kombination mit 1-Hydroxy-2(1H)-Pyridonen (Ciclopirox) und/oder seinen Derivaten und/oder Salzen einen deutlich synergistischen antimykotischen und antiviralen Effekt haben.

Die Erfindung betrifft daher:

Arzneimittel mit synergistischer antimykotischer und antiviraler Wirksamkeit, die dadurch gekennzeichnet sind, daß sie

α) mindestens eine Verbindung, ausgewählt aus:

Ciclopirox und Ciclopiroxderivaten der Formel I

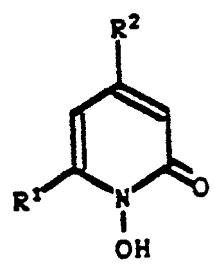

(I)

worin

$R^1$ Wasserstoff, $C_1$-$C_{10}$-Alkyl oder $C_4$-$C_8$-Cycloalkyl und

$R^2$ Wasserstoff oder $C_1$-$C_4$-Alkyl bedeuten

und den physiologisch verträglichen Salzen dieser Verbindungen

und

β) mindestens eine Verbindung, ausgewählt aus:

Nigericin und den Nigericinderivaten der Formel II, III, IV und V

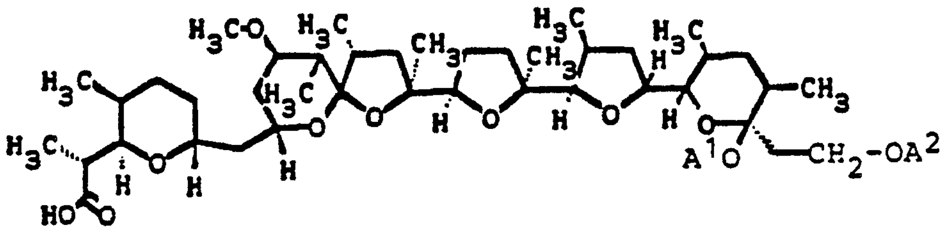

(II)

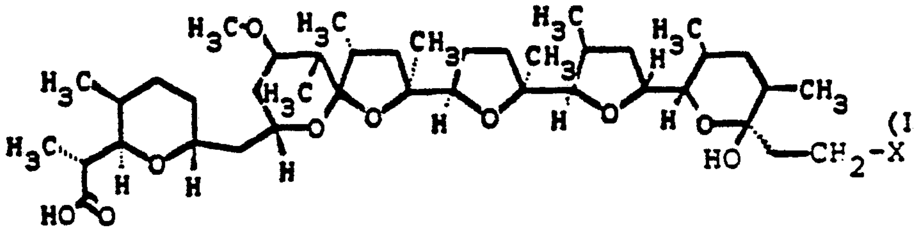

(III)

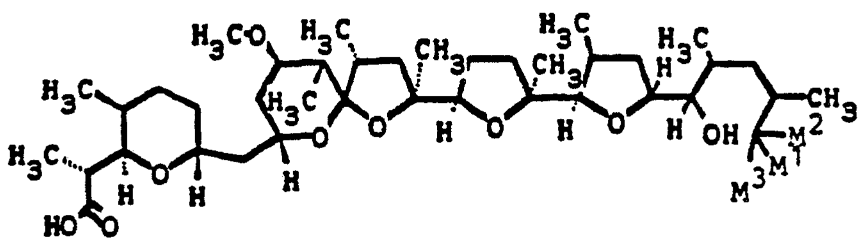

(IV)

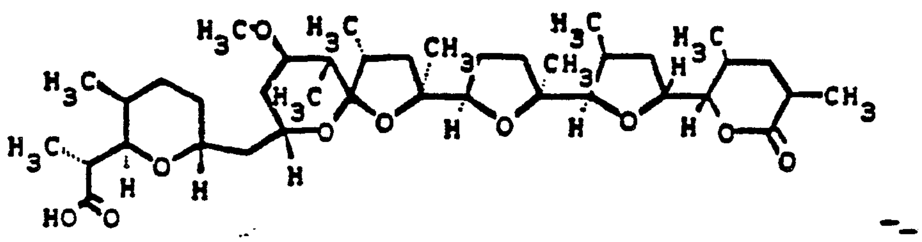

(V)

worin

$A^1$ Wasserstoff oder $C_1$-$C_8$-Alkyl bedeutet und

$A^2$ Wasserstoff bedeutet oder einen Rest der Formel

$-\overset{O}{\overset{\|}{C}}-A^3$, $-\overset{O}{\overset{\|}{C}}-NH-A^3$ oder $-\overset{O}{\overset{\|}{C}}-O-A^3$ darstellt, in der

$A^3$ $C_1$-$C_{15}$-Alkyl bedeutet, welches gegebenenfalls halogen-, nitro-, cyano-, carboxyl-, $C_1$-$C_4$-alkoxy-, phenyloxy oder phenylsubstituiert ist, wobei die genannten Phenylreste ihrereseits mit Halogen, Nitro, Cyano, $C_1$-$C_4$-Alkyl oder $C_1$-$C_4$-Alkoxy substituiert sein können, oder in der

$A^3$ Phenyl, Furyl oder Thienyl bedeutet, wobei diese Phenyl,- Furyl- oder Thienyl-Reste mit Halogen, Nitro, Cyano, $C_1$-$C_4$-Alkyl oder $C_1$-$C_4$-Alkoxy substituiert sein können

oder worin

$A^2$ einen Sulfonsäureester der Formel $-SO_2A^4$ darstellt, worin

$A^4$ Hydroxy, $C_1$-$C_6$-Alkyl, Phenyl oder p-Tolyl bedeutet oder worin

$A^1$ und $A^2$ zusammen einen Rest der Formel VI darstellen

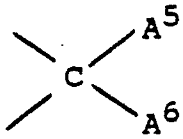

(VI)

in der

$A^5$ und $A^6$ gleich oder verschieden sind und Wasserstoff oder $C_1$-$C_{10}$-Alkyl bedeuten oder wobei

$A^5$ und $A^6$ eine Alkylenkette darstellen, die zusammen mit dem sie tragenden C-Atom einen 5-, 6- oder 7-gliedrigen Ring bildet,

X F, Cl, Br, J, SCN, CN, $NO_2$ oder $N_3$ bedeutet oder einen Substituenten der Formel VII darstellt

Y - $L^1$ (VII)

worin

$L^1$ Wasserstoff, $C_1$-$C_{10}$-Alkyl, $C_2$-$C_{10}$-Alkenyl, $C_3$-$C_8$-Cycloalkyl, Trimethylsilyl, Triethylsilyl, Dimethylphenylsilyl, Dimethylcyclohexylsilyl, Dimethyltertiärbutylsilyl, Diphenyltertiärbutylsilyl, Pyrrolyl, Benzpyrrolyl, Imidazolyl, Benzimidazolyl, Triazolyl, Tetrazolyl oder Phenyl bedeutet, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN mono- oder disubstituiert sind und worin

Y O oder S bedeutet, wobei für $OL^1$ die Bedeutungen Wasserstoff, Pyrrol, Benzpyrrol, Imidazol, Benzimidazol, Triazol und Tetrazol für $L^1$ ausgenommen sind und für

$SL^1$ die Bedeutungen Trimethylsilyl, Triethylsilyl, Dimethylphenylsilyl, Dimethylcyclohexylsilyl, Diphenyltertiärbutylsilyl und Dimethyltertiärbutylsilyl für $L^1$ ausgenommen sind oder worin

X einen Rest der Formel VIII

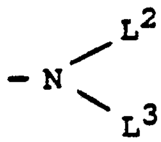

(VIII)

darstellt,

worin

$L^2$ und $L^3$ gleich oder verschieden sind und Wasserstoff, $C_1$-$C_{18}$-Alkyl, $C_2$-$C_{18}$-Alkenyl, $C_3$-$C_8$-Cycloalkyl oder Phenyl bedeuten, wobei die genannten Alkyl-, Alkenyl- und Cycloalkyl-Reste ihrerseits mit Phenyl, Naphthyl oder Thienyl monosubstituiert und die genannten Phenyl- und Alkylreste darüber hinaus noch mit $COOL^4$ monosubstituiert sein können, wobei $L^4$ Wasserstoff oder $C_1$-$C_6$-Alkyl bedeutet oder worin

$L^2$ und $L^3$ zusammen mit dem N-Atom, an dem sie gebunden sind, einen 5- oder 6-gliedrigen N-haltigen Alkylring bilden, wobei eine $CH_2$-Einheit des Rings, welche nicht direkt benachbart zum N-Atom steht, ihrerseits durch NH, O oder S ersetzt sein kann,

$M^1$ Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert ist durch ($C_1$-$C_{10}$)-Alkyl, ($C_1$-$C_{10}$)-Alkoxy oder Phenoxy,

$M^2$ Wasserstoff, ($C_1$-$C_{18}$)-Alkyl (geradkettig oder verzweigt, gesättigt oder ungesättigt), ($C_3$-$C_8$)-Cycloalkyl, Phenyl (unsubstituiert oder substituiert durch ($C_1$-$C_{10}$)-Alkyl, ($C_1$-$C_{10}$)-Alkoxy oder Phenoxy), $CH_2OH$,

$M^3$ OH,

bedeuten,

oder in der

$M^2$ und $M^3$ gemeinsam Sauerstoff und

$M^1$ Wasserstoff, ($C_1$-$C_{18}$)-Alkyl (geradkettig oder verzweigt, gesättigt oder ungesättigt), ($C_3$-$C_8$)-Cycloalkyl, Phenyl (unsubstituiert oder substituiert durch ($C_1$-$C_{10}$)-Alkyl, ($C_1$-$C_{10}$)-Alkoxy oder Phenoxy) oder $NM^4M^5$ bedeuten, worin

$M^4$ und $M^5$ gleich oder verschieden sind und folgende Bedeutungen haben:

i) Wasserstoff,

ii) ($C_1$-$C_{18}$)-Alkyl (gesättigt oder ungesättigt, geradkettig oder verzweigt), wobei die Alkylgruppen unsubstituiert sind oder substituiert mit F, Cl, Br, Phenyl, Naphthyl, Thienyl, $COOM^6$ oder $CON(M^6)_2$ mit $M^6$ gleich Wasserstoff oder ($C_1$-$C_6$)-Alkyl,

iii) ($C_3$-$C_8$)-Cycloalkyl,

iv) Arylgruppen mit insgesamt 6-10 C-Atomen, worin Aryl, Phenyl, Naphthyl, Thienyl bedeutet, welche Systeme entweder unsubstituiert sind oder substituiert mit geradkettigem oder verzweigtem Alkyl mit bis zu 14 C-Atomen, oder F, Cl, Br, J, Nitro, Cyano, Alkoxy, Phenoxy, und den physiologisch verträglichen Salzen der Verbindungen der Formeln II, III, IV und V,

enthalten.

Unter Halogenen werden Fluor, Chlor, Brom und Jod verstanden, insbesondere Chlor und Brom.

Alle genannten Alkyl- oder Alkenylreste mit drei und mehr C-Atomen können sowohl geradkettig als auch verzweigt sein. Unter Aryl und Heteroarylresten werden - soweit nicht gesondert definiert - aromatische oder heteroaromatische Kohlenwasserstoffe verstanden,wie Phenyl, Naphthyl, Pyrrolyl, Imidazolyl, Triazolyl, Tetrazolyl, Furyl oder Thienyl und unter benzokondensierten Heteroarylresten werden benzoannelierte heteroaromatische Kohlenwasserstoffe verstanden, wie Benzpyrrol oder Benzimidazol.

Bei zwei- oder mehrfach substituierten Alkyl-, Alkenyl-, Heteroaryl- oder benzokondensierten Heteroarylresten können die Substituenten sowohl gleich als auch verschieden sein.

Bei den Pyrrolyl-, Benzpyrrolyl-, Imidazolyl-, Benzimidazolyl-, Triazolyl- und Tetrazolyl-Resten handelt es sich bevorzugt um Pyrrol-2-yl, Pyrrol-3-yl, Benzpyrrol-2-yl, Benzpyrrol-3-yl, Imidazol-2-yl, Imidazol-4-yl, Imidazol-5-yl, Benzimidazol-2-yl, 1,2,4-Triazol-3-yl, 1,2,4-Triazol-5-yl oder 1,2,3,4-Tetrazol-5-yl.

Bei den ungesättigten höheren Alkylresten handelt es sich um einfach oder mehrfach ungesättigte Alkylreste, wobei bei mehrfach ungesättigten Alkylen die Doppelbindungen bevorzugt nicht kummuliert sind. Besonders bevorzugt handelt es sich bei den ungesättigten Alkylen um einfach ungesättigte Alkylreste.

Die Herstellung der 1-Hydroxy-2(1H)-pyridone der Formel I ist bekannt und beispielsweise beschrieben in der DE-B 1795270 oder DE-B 2214608, auf die beide an dieser Stelle ausdrücklich Bezug genommen wird. Zur Herstellung werden ungesättigte $\delta$-Ketoester der Formel X oder X′ oder ein Gemisch dieser beiden isomeren Ester

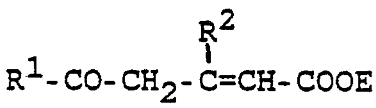
(X)

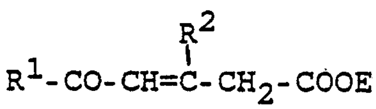
(X')

wobei R¹ und R² die oben zu Formel I angegebenen Bedeutungen haben und E eine nieder molekulare Alkylgruppe darstellt, mit Hydroxylamin oder dessen Salzen unter sauren oder alkalischen Bedingungen umgesetzt. Die als Ausgangssubstanzen verwendeten $\delta$-Ketoestern lassen sich beispielsweise durch Kondensation von Carbonsäurechloriden mit $\beta,\beta$-Dialkyl-acrylsäureestern in Gegenwart von Friedel-Crafts-Katalysatoren gewinnen.

Ein alternatives Herstellungsverfahren beruht auf der Umsetzung von 2-Pyronen der Formel XI

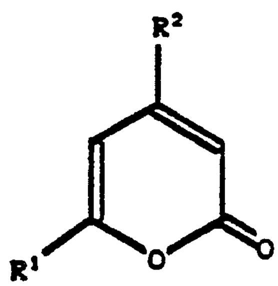
(XI)

worin $R^1$ und $R^2$ die oben zu Formel I angegebenen Bedeutungen haben,
mit Hydroxylamin oder einem seiner Salze in Gegenwart eines gegebenenfalls in der Aminogruppe oder im Ring alkylsubstituierten Aminopyridins oder Imidazols. Die als Ausgangssubstanzen verwendeten 2-Pyrone sind nach verschiedenen Verfahren leicht zugänglich (z.B. R.C. Elderfield, Heterocyclic Compounds, 2. Aufl., Vol. 1, S. 354 ff, J. Wiley and Sons, Inc., New York 1959; Chem. Ber. 100, S. 658 ff. (1967)).

Die Verbindungen der Formel II können -wie bereits in der deutschen Patentanmeldung P 38oo598.0 vorgeschlagen-beispielsweise hergestellt werden indem man

a) Nigericin (Formel II: $A^1 = A^2 = H$) oder ein $A^1$-Derivat umsetzt mit einer Verbindung der Formel XII oder XIII

$Z-\overset{\overset{O}{\|}}{C}-A^3$ (XII) $Z-\overset{\overset{O}{\|}}{C}-O-A^3$ (XIII)

worin
Z Chlor, Brom oder ein Anhydrid bedeutet und
$A^3$ die oben zu Formel II angegebenen Bedeutungen hat,
oder indem man

b) Nigericin oder ein $A^1$-Derivat umsetzt mit einem Isocyanat der Formel XIV

$O = C = N - A^3$ (XIV)

worin $A^3$ die oben zu Formel II angegebenen Bedeutungen hat,
oder indem man

c) Nigericin oder ein $A^1$-Derivat umsetzt mit einem $SO_3$/Pyridin-Komplex oder mit einer Verbindung der Formel $G-SO_2-A^4$, wobei G Chlor oder Brom bedeutet und $A^4$ bis auf Wasserstoff, die oben zu Formel II angegebenen Bedeutungen hat,
oder indem man

d) Nigericin oder ein $A^2$-Derivat in Gegenwart einer Lewis-Säure umsetzt mit einem Alkohol der Formel XV

$A^1 - OH$ (XV)

worin $A^1$ bis auf Wasserstoff die oben zu Formel II angegebenen Bedeutungen hat,
oder indem man

e) Nigericin in Gegenwart eines Katalysators umsetzt mit einer Carbonylverbindung der Formel XVI

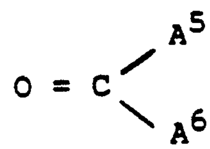

(XVI)

worin $A^5$ und $A^6$ die oben zu Formel II angegebenen Bedeutungen haben.

Unter einem $A^1$- bzw. $A^2$-Derivat werden solche Verbindungen der Formel II verstanden, bei denen $A^2$ (= $A^1$-Derivat) bzw. $A^1$ (= $A^2$-Derivat) Wasserstoff bedeutet und der jeweils andere Rest von Wasserstoff verschieden ist. Unter Lewis-Säuren werden hier Kupfer-, Eisen- oder Lithiumhalogenide wie Kupferchlorid, Eisen(III)-chlorid oder Lithiumbromid, insbesondere Lithiumbromid, verstanden.

Mit Hilfe der Verfahrensvarianten a), b) und c) lassen sich unterschiedliche Substituenten $A^2$ in das Molekül einführen. Mit Hilfe der Verfahrensvarianten d) läßt sich der Substituent $A^1$ in das Molekül einführen. Verfahrensvariante e) dient zur Herstellung solcher Nigricinderivate, in denen $A^1$ und $A^2$ zusammen einen Rest der Formel VI darstellen.

Die Reihenfolge der Derivatisierung ist im allgemeinen unkritisch, d. h. es kann sowohl zunächst der Substituent $A^2$ und gegebenenfalls anschließend der Substituent $A^1$ in das Molekül eingeführt werden, als auch umgekehrt, also zunächst $A^1$ und dann $A^2$.

Bei der Verfahrensvarianten a) geht man am besten so vor, daß man Nigericin oder ein $A^1$-Derivat in äquimolaren Mengen oder in bis zu einem 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel XII oder XIII - insbesondere den Anhydriden - bis zur Beendigung der Reaktion umsetzt, gegebenenfalls in Gegenwart einer Base, vorzugsweise Pyridin oder DMAP (Dimethylaminopyridin).

Die Reaktionstemperaturen liegen dabei zwischen $-70^\circ C$ und $+100^\circ C$, vorzugsweise bei Verwendung eines Lösungsmittels zwischen dem Festpunkt und dem Siedepunkt des Lösungsmittels, insbesondere zwischen $-70^\circ C$ und $+40^\circ C$. Die Reaktionszeiten betragen 1 bis 180 Stunden, bevorzugt 1 bis 48 Stunden. Die Beendigung der Reaktion kann beispielsweise mittels Dünnschichtchromatographie bestimmt werden (DC-Kontrolle).

Das als Ausgangssubstanz benötigte Nigericin kann beispielsweise nach dem von J. Berger (J. Berger et al., Am. Chem. Soc., 73, 5295 (1951)) beschriebenen Verfahren hergestellt werden. Es kann aber auch gemäß dem in der deutschen Patentanmeldung P 3700325.9 vorgeschlagenen Verfahren hergestellt werden,
wobei Nigericin neben Amycin bei der Kultivierung von Streptomyces parvulus DSM 3816 entsteht. Das Nigericin kann aus dem Mycel mittels Hexan extrahiert und nach Aufkonzentrierung auskristallisiert werden.

Die Ausgangsverbindungen für die Verfahrensvariante a), die Verbindungen der Formel XII oder XIII sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter der Gruyter, Berlin, New York 1982, S. 303 ff. (Zur Herstellung der Anhydride siehe beispielsweise: ORGANIKUM, Organisch-Chemisches Grundpraktikum, 15. Auflage (1976), VEB Deutscher Verlag der Wissenschaften, Berlin, Methodenregister S. 824: Carbonsäureanhydride).

Bei der Verfahrensvarianten b) geht man am besten so vor, daß man das Nigericin oder ein $A^1$-Derivat in äquimolaren Mengen oder in einem bis zu 50-fachen Überschuß umsetzt, mit einer Verbindung der Formel XIV.

Gegebenenfalls kann auch diese Umsetzung unter Basenzusatz erfolgen. Als Basen kommen beispielsweise Triethylamin, Pyridin oder Lutidin in Frage. Eine Variante des Verfahrens b) besteht darin, daß man in einem geeigneten, vorzugsweise inerten Lösungsmittel wie Chloroform, Methylenchlorid, THF, Essigester oder Dioxan arbeitet. Auch hier kann der Überschuß der oben aufgeführten Verbindungen bis zur 50-fachen Menge betragen.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 180 Stunden, bevorzugt 1 bis 48 Stunden. Die Beendigung der Reaktion kann beispielsweise mittels DC-Kontrolle bestimmt werden.

Die Ausgangsverbindungen für die Verfahrensvariante b) sind, sofern nicht käuflich, auf einfache Weise nach literaturbekannten Verfahren herstellbar (Isocyanate: Houben-Weyl, 4. Auflage, Georg Thieme Verlag Stuttgart (1983), Band E4).

Bei der Verfahrensvarianten c) geht man am besten analog der Verfahrensvarianten b) vor. Der $SO_3$/Pyridin-Komplex ist käuflich erhältlich. Die Aryl- bzw. Alkylsulfonsäurehalogenide der Formel G-$SO_2A^4$ erhält man beispielsweise durch radikalische Umsetzung von Alkanen mit Chlor und $SO_2$ oder durch Halogenierung von Aromaten mit Halogensulfonsäure G-$SO_3H$.

Bei der Verfahrensvarianten d) geht man am besten so vor, daß man Nigericin oder ein entsprechendes $A^2$-Derivat mit einem Überschuß an einem Alkohol der Formel $A^1$-OH in Gegenwart einer Lewissäure bis zur Beendigung der Reaktion umsetzt. Eine Variante des Verfahrens besteht darin, daß man in einem geeigneten Lösungsmittel wie Chloroform, Methylenchlorid, THF, Essigester oder Dioxan arbeitet. Als Lewissäure eignen sich beispielsweise Kupfer-, Eisen- oder Lithium-Halogenide, insbesondere $CuCl_2$, $FeCl_3$ oder LiBr. Ganz besonders bevorzugt ist die Verwendung von Lithiumbromid.

Die Konzentration der Lewissäure - bezogen auf Nigericin oder das Nigericinderivat - beträgt 0,1 bis 50 Gew.-%, bevorzugt 0,5 bis 20 Gew.-%. Die Reaktionstemperaturen liegen dabei zwischen -40 °C und +100 °C, insbesondere zwischen 0 °C und 30 °C, vorzugsweise bei Verwendung eines Lösungsmittels zwischen dem Festpunkt und dem Siedepunkt des Lösungsmittels, insbesondere zwischen 0 °C und 30 °C. Die Reaktionszeiten betragen 1 bis 180 Minuten, bevorzugt 5 bis 60 Minuten. Die Beendigung der Reaktion kann beispielsweise mittels Dünnschichtchromatographie bestimmt werden.

Bei der Verfahrensvarianten e) geht man am besten so vor, daß man Nigericin im Überschuß mit einer Verbindung der Formel XVI bis zur Beendigung der Reaktion umsetzt, gegebenenfalls in Gegenwart eines Katalysators wie $ZnCl_2$, $ZnBr_2$, $FeCl_3$, $CuSO_4$ oder $CuCl_2$. Die Konzentration dieses Katalysators - bezogen auf Nigericin - beträgt vorzugsweise 0,1 bis 50 %. Eine Variante des Verfahrens besteht darin, daß man in einem geeigneten aprotischen Lösungsmittel wie Chloroform, Methylenchlorid, THF, Essigester oder Dioxan arbeitet. Auch hier kann ein Überschuß an XVI angewandt werden.

Die Reaktionstemperaturen liegen dabei zwischen -20 °C und +100 °C, vorzugsweise bei Verwendung eines Lösungsmittels zwischen Raumtemperatur und dem Siedepunkt des Lösungsmittels, insbesondere zwischen 20 °C und 100 °C. Die Reaktionszeiten betragen 1 bis 50 Stunden, bevorzugt 1 bis 12 Stunden. Die Beendigung der Reaktion kann beispielsweise mittels DC-Kontrolle bestimmt werden.

Die Ausgangsverbindungen für die Verfahrensvariante e), die Verbindungen der Formel XVI sind, sofern nicht käuflich, auf einfache Weise nach literaturbekannten Verfahren herstellbar. Beispielsweise erhält man Aldehyde der Formel XVI durch Oxidation von primären Alkoholen oder durch Reduktion von Carboxylderivaten, beispielsweise mit komplexen Hydriden oder durch Reduktion von Carbonsäurechloriden (weitere Beispiele zur Synthese von Aldehyden bzw. Ketonen finden sich in Organikum, Organisch Chemisches Praktikum, 15. Auflage, VEB Deutscher Verlag der Wissenschaften, Berlin 1976, Methodenregister, Aldehyde, Ketone, S. 819 ff.).

Die Reinigung, Isolierung und Aufarbeitung der Substanzen erfolgt nach den üblichen Methoden; beispielsweise können die Reaktionsprodukte durch Chromatographie an polaren Trägermaterialien wie

Kieselgel oder ®Sephadex LH 20 mit Lösungsmitteln wie niederen Alkanolen wie Methanol oder Chloroform oder Essigester oder Methanol/Chloroform-Mischungen, aber auch extraktive Methoden wie flüssig/flüssig-Extraktion oder fest/flüssig-Extraktion oder durch Kristallisation gereinigt werden.

Die Verbindungen der Formel III können -wie bereits in der deutschen Patentanmeldung P 3820179.8 vorgeschlagen-beispielsweise hergestellt werden, in dem man Nigericin mit einer einen nukleofugen Rest liefernden Verbindung XVII

Hal - Q (XVII)

worin

Hal Chlor, Brom oder Jod und

Q $-SO_2CH_3$, $-SO_2CF_3$,

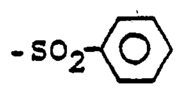

oder

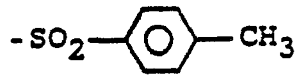

bedeuten,

zu einer Verbindung der Formel XVIII

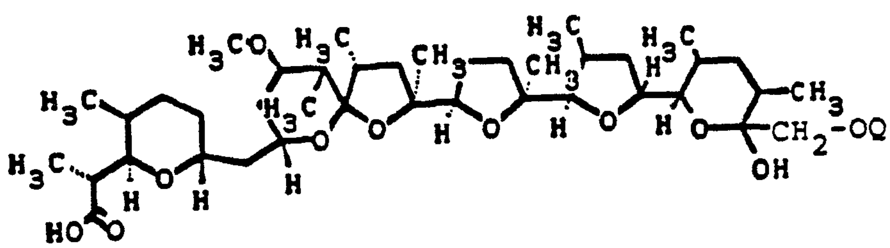 (XVIII)

wobei Q die oben zu Formel XVII angegebenen Bedeutungen hat, umsetzt,

und anschließend

f) die Verbindung XVIII mit $NaN_3$ oder einer Verbindung der Formel XIX umsetzt,

Met. - X′ (XIX)

worin

Met. Li, Na, K oder ein quartäres, organisches Ammoniumfragment bedeutet und

X′ F, Cl, Br, J, SCN, CN oder $NO_2$ bedeutet, wobei man eine Verbindung der Formel III′ erhält,

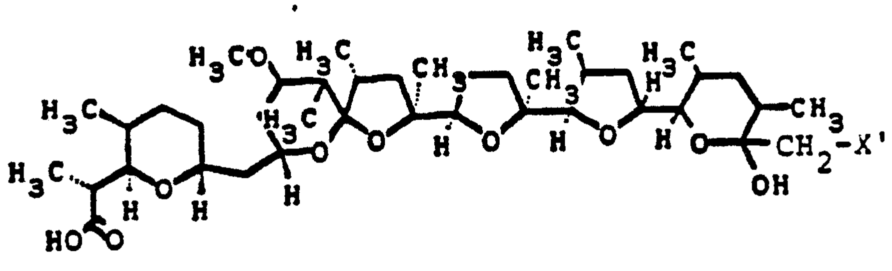 (III′)

worin

X′ $N_3$ ist oder die oben zu Formel XIX angegebenen Bedeutung hat

oder daß man

g) die Verbindung der Formel XVIII mit einem Alkohol, Thiol oder Amin der Formel XX bzw. XX′ umsetzt,

HY - $L^1$ (XX)

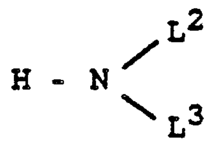

(XX')

worin

Y, $L^2$ und $L^3$ die oben zu Formel III angegebenen Bedeutungen haben und

$L^1$ bis auf Trimethylsilyl, Triethylsilyl, Dimethylphenylsilyl, Dimethylcyclohexylsilyl, Diphenyltertiärbutylsilyl und Dimethyltertiärbutylsilyl

die oben zu Formel III angegebenen Bedeutungen hat, wobei man eine Verbindung der Formel III erhält, in der X Y-$L^1$ oder

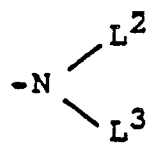

bedeutet, wobei Y, $L^2$ und $L^3$ die oben zu Formel III angegebenen Bedeutungen haben und $L^1$ bis auf Trimethylsilyl, Triethylsilyl, Dimethylphenylsilyl, Dimethylcyclohexylsilyl, Diphenyltertiärbutylsilyl und Dimethyltertiärbutylsilyl die oben zu Formel III angegebenen Bedeutungen hat

oder daß man

h) Nigericin umsetzt mit einer Verbindung der Formel XXI

Hal′ - Sil (XXI)

worin

Hal′ Chlor oder Brom und

Sil Trimethylsilyl, Triethylsilyl, Dimethylphenylsilyl, Diemethylcyclohexylsilyl, Diphenyltertiärbutylsilyl oder Dimethyltertiärbutylsilyl bedeutet,

wobei man eine Verbindung der Formel III erhält, in der

X Y-$L^1$ bedeutet, wobei Y Sauerstoff ist und

$L^1$ Trimethylsilyl, Triethylsilyl, Dimethylphenylsilyl, Dimethylcyclohexylsilyl, Diphenyltertiärbutylsilyl oder Dimethyltertiärbutylsilyl bedeutet.

Unter einem quartären organischen Ammoniumfragment werden die kationischen quartären Reste von Ammoniumsalzen verstanden, wie Tetraphenylammonium oder Tetrabutylammonium. Als geeignete Salze seien beispielsweise genannt: Tetrabutylammoniumchlorid, Tetrabutylammoniumbromid oder Tetraphenylammoniumcyanid.

Unter einem nukleofugen Rest werden solche Substituenten verstanden, die bei einer nukleophilen Substitution leicht vom betreffenden Molekül abgespalten werden können und somit den Eintritt eines nukleophilen Agens erleichtern bzw. erst möglich machen. Gängige nukleofuge Gruppen sind beispielsweise Cl, Br, J, Methylsulfonate, Phenylsulfonate oder Toluolsulfonate.

Mit Hilfe der Verfahrensvarianten f) und g) lassen sich sämtliche Substituenten, bis auf die Silylverbindungen, beispielsweise durch nukleophile Substitution des Sulfonsäurederivats in das Molekul einführen. Die Einführung der Silylsubstituenten gemäß Verfahrensvariante h) gelingt durch einfache Umsetzung von Nigericin (Formel III : X = OH) mit den entsprechenden Silylhalogeniden, bevorzugt -chloriden.

Zur Herstellung der Sulfonsäurederivate geht man am besten so vor, daß man Nigericin (Formel III: X = OH) in äquimolaren Mengen oder in einem bis zu 50-fachen Überschuß mit einem Sulfonsäurehalogenid, bevorzugt -chlorid, besonders bevorzugt Methylsulfonylchlorid, umsetzt. Gegebenenfalls kann diese Umsetzung unter Basenzusatz erfolgen. Als Basen kommen beispielsweise Triethylamin, Pyridin oder Lutidin in Frage. Eine Variante des Verfahrens besteht darin, daß man in einem geeigneten, vorzugsweise inerten Lösungsmittel wie Chloroform, Methylenchlorid, Tetrahydrofuran (THF), Essigester oder Dioxan arbeitet. Auch hier kann der Überschuß an Sulfonsäurehalogenid bis zur 50-fachen Menge betragen.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C.

Die Reaktionszeiten betragen 1 bis 180 Stunden; bevorzugt 1 bis 48 Stunden, besonders bevorzugt 1 bis 8 Stunden. Die Beendigung der Reaktion kann beispielsweise mittels dünnschichtchromatographischer Kontrolle (DC-Kontrolle) bestimmt werden.

Die als Ausgangssubstanzen verwendeten Sulfonsäurehalogenide sind käuflich.

Bei der Verfahrensvarianten f) geht man am besten so vor, daß man das Sulfonsäurederivat der Formel XVIII mit Natriumazid oder mit dem Salz der Formel XIX in äquimolaren Mengen oder in einem bis zu 50-fachen Überschuß des Salzes in einem inerten Lösungsmittel wie Dimethylformamid (DMF), Tetrahydrofuran (THF), Aceton, Acetonitril, Toluol, Dimethylsulfoxid (DMSO) oder Chloroform umsetzt. Bevorzugt werden die Lithium-, Natrium- oder Kaliumsalze, besonders bevorzugt die quartären organischen Ammoniumsalze der Formel XIX eingesetzt. Bevorzugte Lösungsmittel bei der Umsetzung mit den Ammoniumsalzen sind THF, Chloroform und Toluol. Bei der Umsetzung mit $NaN_3$ ist DMF als Lösungsmittel bevorzugt.

Die Reaktionstemperaturen liegen bei dieser Umsetzung zwischen -70° C und dem Siedepunkt des Lösungsmittels, insbesondere bei -70 bis 100° C, bevorzugt bei 20 bis 90° C, insbesondere bei der Umsetzung mit den quartären Ammoniumsalzen bei 60° C.

Die Reaktionszeiten betragen 10 bis 100 Stunden, bevorzugt 10 bis 60 Stunden. Die Beendigung der Reaktion kann beispielsweise mittels DC-Kontrolle bestimmt werden.

Die als Ausgangsverbindungenen eingesetzten Lithium-, Natrium-, Kalium-, -Fluoride, -Chloride, -Bromide, -Jodide, -Rhodanide, -Cyanide oder -Nitrite sind käuflich; ebenso die quartären organischen Ammoniumsalze wie Tetraphenyl- oder Tetrabenzyl-ammoniumchlorid, -bromid oder -rhodanid.

Die Herstellung der erfindungsgemäßen Verbindungen nach Verfahrensvariante g) gelingt am einfachsten dadurch, daß die beiden Komponenten, das Nigericinsulfonat XVIII und das entsprechende Amin, Thiol oder der Alkohol gemäß Formel XX bzw. XX′ in äquimolaren Mengen oder in bis zu einem 5-fachen Überschuß an XX bzw. XX′ vermengt werden und bei Temperaturen zwischen 50 und 210° C, bevorzugt bei 60 - 120° C, bis zur Beendigung der Reaktion erhitzt werden. Die Beendigung der Reaktion läßt sich mittels Dünnschichtchromatographie bestimmen. Die Reaktionszeiten betragen 1 - 200 Stunden, bevorzugt 10 - 60 Stunden.

Eine Variante des Verfahrens g) besteht darin, daß man in einem geeigneten Lösungsmittel, wie Diethylether oder Dimethoxyethan oder THF, Alkoholen, wie Methanol, Ethanol oder Methylglykol, chlorierten Kohlenwasserstoffen, wie Methylenchlorid, Chloroform, Tri- oder Tetrachlorethylen, Toluol, Benzol oder auch polaren Lösungsmitteln wie DMF oder DMSO arbeitet. Sofern die Amine, Thiole oder Alkohole, die umgesetzt werden sollen, flüssig sind, können diese selbstverständlich auch als Lösungsmittel eingesetzt werden. Auch bei der Lösungsmittelvariante kann ein Überschuß von Amin, Thiol oder Alkohol gemäß Formel XX bzw. XX′, der bis zur etwa 5-fachen Menge betragen kann, angewandt werden. Die Reaktionstemperaturen liegen dabei zwischen dem Festpunkt und dem Siedepunkt des Lösungsmittels, insbesondere zwischen -70° C und +40° C.

Die Reinigung, Isolierung und Aufarbeitung der Substanzen erfolgt nach den üblichen Methoden.

Die Verbindungen der Formel IV werden -wie bereits in der deutschen Patentanmeldung P 3811016.4 vorgeschlagen- nach einem Verfahren erhalten, bei welchem man (Verfahrensvariante j) Nigericin mit einer Verbindung der Formel $M^1MgHal$ umsetzt, in welcher $M^1$ Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert durch ($C_1$-$C_{10}$)-Alkyl, ($C_1$-$C_{10}$)-Alkoxy oder Phenoxy, und Hal Chlor, Brom oder Iod bedeuten.

Ein weiteres Verfahren zum Herstellen der Verbindungen der Formel IV besteht darin, daß man (Verfahrensvariante k) Nigericin mit Natriumperiodat zu Verbindungen der Formel V

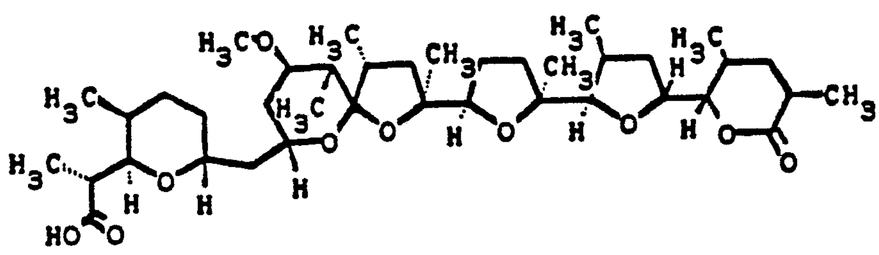

(V)

spaltet und diese Verbindung V analog zu der vorher beschriebenen Verfahrensvariante (j) mit $M^1MgHal$ zu Verbindungen der Formel IV umsetzt.

Die Verbindung IV mit $M^1$ = Wasserstoff, $M^2$ = Wasserstoff und $M^3$ = OH kann (Verfahrensvariante l) aus der erwähnten Verbindung V durch Reduktion mit Hydridüberträgern erhalten werden.

Verbindungen der Formel IV, in denen $M^2$ und $M^3$ gemeinsam Sauerstoff bedeuten, lassen sich (Verfahrensvariante m) nach einem Verfahren herstellen, bei welchem Verbindungen der Formel V, wie beschrieben, hergestellt werden, und bei welchem anschließend diese Verbindungen der Formel V mit Aminen $HNM^4M^5$ mit $M^4$ und $M^5$, wie oben definiert, umgesetzt werden.

Verbindungen der Formel IV mit $M^1$ = Wasserstoff, ($C_1$-$C_{18}$)-Alkyl (geradkettig oder verzweigt, gesättigt

oder ungesättigt), ($C_3$-$C_8$)-Cycloalkyl, Phenyl (unsubstituiert oder substituiert durch ($C_1$-$C_{10}$)-Alkyl, ($C_1$-$C_{10}$)-Alkoxy oder Phenoxy),
und mit $M^2$ und $M^3$ gemeinsam gleich Sauerstoff werden (Verfahrensvariante n) erhalten durch Natriumperiodat-Spaltung von Verbindungen der Formel IV mit $M^1$ wie soeben definiert und $M^2$ = $CH_2OH$ und $M^3$ = OH.

Diese Verbindungen der Formel IV mit $M^2$ und $M^3$ gemeinsam gleich Sauerstoff liegen im Gleichgewicht mit ihrer cyclischen Hemiacetalform IVa vor:

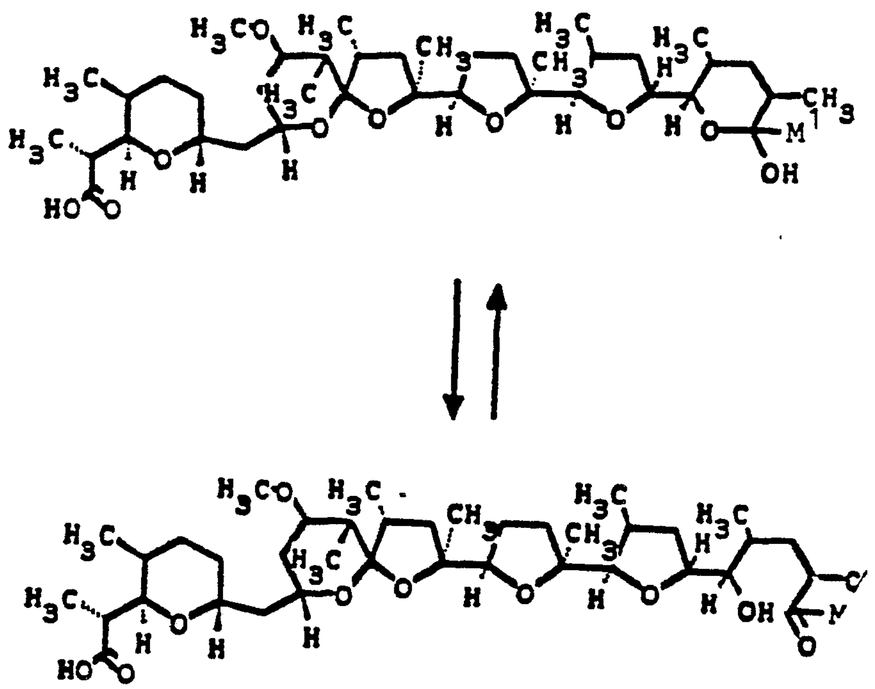

(IVa)

(IV)

Bei der Verfahrensvariante j) geht man am besten so vor, daß man Nigericin oder das Lacton V mit einem Überschuß einer Grignard-Verbindung der Formel $M^1MgHal$ in einem inerten Lösungsmittel, bevorzugt in einem Ether, wie Tetrahydrofuran, Dioxan oder Diethylether, bis zur Beendigung der Reaktion umsetzt, gegebenenfalls in Gegenwart von Magnesiumhalogeniden, vorzugsweise Magnesiumchlorid.

Die Reaktionstemperaturen liegen dabei zwischen -70° C und +70° C, insbesondere zwischen -70° C und +40° C.

Die Reaktionszeiten betragen 1-180 Stunden, bevorzugt 10-48 Stunden. Die Beendigung der Reaktionen kann beispielsweise mit Hilfe der Dünnschichtchromatographie festgestellt werden.

Die Ausgangsverbindungen für die Verfahrensvariante j), die Grignard-Verbindungen der Formel $M^1MgHal$, sind, sofern sie nicht käuflich sind, auf einfache Weise nach literaturbekannten Verfahren herstellbar. Beispielsweise erhält man die Grignard-Verbindungen durch Umsetzung der entsprechenden Alkyl- oder Arylhalogenide mit Magnesium in Ether.

Bei der Verfahrensvariante k) geht man am besten so vor, daß man Nigericin mit einem Überschuß an Natriumperiodat nach bekannten Verfahrensweisen zu der Verbindung der Formel V umsetzt. Als Lösungsmittel dienen die für die Spaltung mit Natriumperiodat üblichen Lösungsmittel, die literaturbekannt sind; z.B. eignen sich Alkohole oder fast alle anderen mit Wasser mischbaren Lösungsmittel. Bevorzugt ist ein Gemisch aus Tetrahydrofuran und Wasser.

Die weitere Umsetzung mit der Grignard-Verbindung nach Variante k) erfolgt wie oben für Variante j) beschrieben.

Nach Verfahrensvariante l) setzt man vorteilhaft die Verbindung V mit einem Überschuß an Hydridüberträgern um.

Neben Lithiumaluminiumhydrid sind Natriumborhydrid und Natriumcyanoborhydrid bei erhöhten Temperaturen sehr geeignet.
Geeignet Lösungsmittel sind besonders Ether, wie Tetrahydrofuran, Dioxan oder Diethylether. Die Reaktionsbedingungen für die Reduktion mit dem Hydridüberträger werden so gewählt, daß die Reaktion ausschließlich bzw. ganz überwiegend am sogenannten F-Ring des Nigericins stattfindet, und daß die

Carboxylgruppe auf der anderen Seite des Moleküls (am A-Ring) unverändert bleibt. Als geeignet für diese Führung des Reaktion haben sich Temperaturen von -30 bis +40 °C erwiesen, insbesondere solche von 0 bis 20 °C. Die Reaktionszeiten liegen je nach Reaktionstemperaturen zwischen 1 und 60 Stunden, bevorzugt zwischen 4 und 12 Stunden. Ganz besonders geeignet ist Lithiumalanat als Reduktionsmittel. Im übrigen sind Reduktionen mit Hydridüberträgern literaturbekannte Reaktionen.

Nach Variante m) stellt man, wie oben beschrieben, zunächst die Verbindung V her und setzt diese dann mit einem Amin $HNM^4M^5$ um. Vorteilhaft ist hierbei ein bis zu 50-facher Überschuß des Amins als Lösungsmittel, oder man setzt ein inertes Lösungsmittel ein, z.B. Chloroform, Methylenchlorid, Tetrahydrofuran oder Dioxan. Vorteilhaft ist es auch, die Reaktion in Gegenwart einer Base durchzuführen. Als besonders vorteilhaft hat es sich erwiesen, die Gesamtreaktion in Tetrahydrofuran auszuführen; als zuzusetzende Base ist Dimethylaminopyridin besonders geeignet. Die Reaktionstemperaturen liegen zwischen -70 °C und +70 °C, bevorzugt zwischen 0 und 30 °C. Die Raktionszeiten liegen zwischen 5 und 20 Stunden.

Bei der Verfahrensvariante n) geht man so vor, daß man die Verbindungen, die nach Verfahrensvariante j) durch Addition von Grignard-Verbindungen an Nigericin entstehen, nach Verfahrensvariante k) mit Natriumperiodat spaltet.

Verbindung V

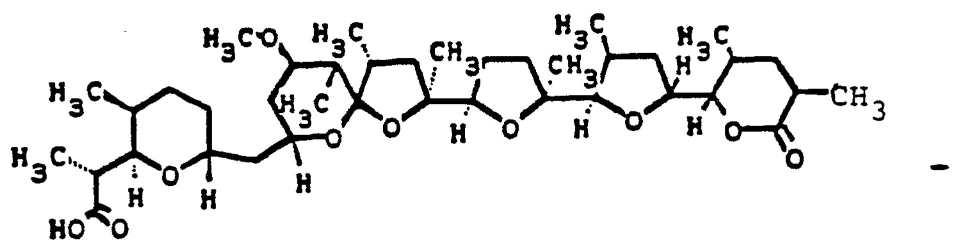

(V)

wird hergestellt wie in Verfahrensvariante k) beschrieben.

Die Reinigung, Isolierung und Aufarbeitung der Substanzen erfolgt nach den üblichen Methoden. Die physiologisch verträglichen Salze der Verbindungen der Formeln I, II, III, IV und V können nach literaturbekannten Verfahren aus eben diesen Verbindungen hergestellt werden.

Die erfindungsgemäße Kombination von Ciclopiroxen und Nigericinen wirkt stark antibakteriell, antimykotisch und antiviral und ist daher zur Behandlung von Infektionen besonders gut geeignet. Wesentlich ist die Tatsache, daß die Wirkung der beiden Komponenten gegen Pilze und Viren sich nicht nur additiv verhält, sondern vielmehr ein unerwarteter, stark synergistischer Effekt auftritt. Selbst bei Pilzen, gegen die Nigericine selbst keine signifikate antimykotische Wirkung haben, ist in der erfindungsgemäßen Kombination eine deutliche Steigerung der antimykotischen Wirkung der Ciclopiroxe bei Zusatz von Nigericin zu beobachten.

Die erfindungsgemäßen Zubereitungen erfassen somit ein Keimspektrum bei niedrigen minimalen Hemmkonzentrationen, die von den Einzelkomponenten nicht erreicht werden.

Aus den genannten Gründen ist die erfindungsgemäße Zubereitung den Einzelkomponenten bei der Behandlung von Infektionen überlegen. Sie erlauben es, geringere Dosierungen der Einzelkomponenten zuzuführen und dennoch einen besseren Therapieerfolg zu erzielen.

Die Erfindung betrifft auch ein Verfahren zur Herstellung einer solchen Zubereitung, das dadurch gekennzeichnet ist, daß man

α) Ciclopirox und/oder mindestens ein Ciclopiroxderivat und/oder ein oder mehrere physiologisch verträgliche Salze dieser Verbindungen (Komponente A) und

β) Nigericin und/oder mindestens ein Nigericinderivat und/oder ein oder mehrere physiologisch verträgliche Salze dieser Verbindungen (Komponente B) gegebenenfalls zusammen mit einem oder mehreren physiologisch annehmbaren Träger und gegebenenfalls weiteren Hilfs- oder Zusatzstoffen in eine geeignete Darreichungsform bringt. Bevorzugt ist die topische Darreichungsform.

Die Erfindung betrifft weiterhin ganz allgemein pharmazeutische Erzeugnisse (Arzeimittel), die nebeneinander, in vermischter oder unvermischter Form

α) Ciclopirox und/oder mindestens ein Ciclopiroxderivat und/oder ein oder mehrere physiologisch verträgliche Salze dieser Verbindungen (Komponente A) und

β) Nigericin und/oder mindestens eine Nigericinderivat und/oder ein oder mehrer physiologisch verträgliche Salze dieser Verbindungen (Komponente B) als Kombinationspräparat zur gleichzeitigen, getrennten oder zeitlich abgestuften Anwendung zur - bevorzugt topischen - Behandlung bakterieller,

mykotischer und viraler Infektionen enthalten.

Das Verhältnis der Einzelkomponenten in der Kombination kann zwischen etwa Komponente A : Komponente B = 1 : 9 und Komponente A : Komponente B = 9 : 1, vorzugsweise zwischen etwa Komponente A : Komponente B = 1 : 5 und Komponente A : Komponente B = 5 : 1, liegen. Die Dosis in einer Verabreichungseinheit (Summe der Komponenten A und B) kann zwischen 10 und 50 mg gewählt werden. Diese Dosierungen können in schweren Fällen auch erhöht werden. In den meisten Fällen genügen jedoch auch geringere Dosierungen.

Da die Herstellung der Ciclopiroxkomponente im allgemeinen weniger aufwendig und damit auch preiswerter ist als die Herstellung der Nigericin-Komponente, wird man schon aus diesem Grund eine erfindungsgemäße Kombination vorziehen, in der der Anteil der Ciclopirox-Komponente höher liegt als der der Nigericin-Komponente.

Die erfindungsgemäßen Zubereitungen bzw. Erzeugnisse können in verschiedenen Dareichungsformen verabreicht werden, wobei die topische bevorzugt ist.

Die chemotherapeutisch verwendbaren, erfindungsgemäßen Kombinationen der Wirkstoffe können zur Herstellung von pharmazeutischen Präparaten verwendet werden, welche eine wirksame Menge der Aktivsubstanzen, zusammen mit üblichen Hilfs- und/oder Trägerstoffen enthalten. Für die bevorzugte topische Verabreichungsform können dies Lösungen, Salben, Cremes, Puder, oder Suspensionen sein, die gegebenenfalls sterilisiert sind und gegebenenfalls Hilfs- und/oder Trägerstoffer, wie Konservier-, Stabilisierungs-, Netz-und/oder Emulgiermittel, Löslichkeitsvermittler, Salze zur Regulierung des osmotischen Drucks und/oder Puffersubstanzen enthalten. Auch eine Einarbeitung der erfindungsgemäßen Wirkstoffkombination in topisch anwendbare Träger, wie Wundauflagen, Folien, Gele und Lacke ist möglich.

Die folgenden Beispiele dienen zur Erläuterung der Erfindung.

Beispiele:

**Synergistische Wirkung der Wirkstoffkombination Cyclopiroxolamin (= Ethanolamin des 6-Cyclohexyl-1-Hydroxy-4-methyl-2(1H)-pyridon) mit verschiedenen Nigericinderivaten gegen die Teststämme Microsporum Canis und Candida albicans.**

Die minimale Hemmkonzentration (MHK), das heißt, diejenige Konzentration bei der das Wachstum der Pilze noch makroskopisch sichtbar gehemmt ist, wurde im Mitrkoverdünnungstest (Mikrodilutiontest in Neopepton Dextrose Boullion, siehe: "Comparison of two different techniques in primary micrological screening": Standard serial dilution test and microtitration test; W. Raether, M. Uphoff und G. Staut, mycosen, 27 (1984), p. 15 - 28) geprüft. Das Inokulum pro Well betrug bei Candida albicans $10^3$ cfu/ml und bei Microsporum canis $1,6 \cdot 10^5$ cfu/ml (cfu = colony forming units).

Zunächst wurde die minimale Hemmkonzentration eines jeden Kombinationspartners (Ciclopiroxolamin bzw. Nigericin oder Nigericinderivat) allein festgestellt (Cyclopyoxolamin: 2 µg/ml; Nigericin und alle Nigericinderivate: >128 µg/ml). Anschließend wurde die MHK für Ciclopiroxolamin in Anwesenheit von jeweils 64 µg/ml, 16 µg/ml und 1 µg/ml eines Nigericinderivats geprüft, um festzustellen inwieweit der Zusatz des Nigericinderivats die Wirkung von Ciclopiroxolamin verstärken konnte.

In der Tabelle 1 sind die Formeln der verwendeten Nigericinderivate aufgeführt und in Tabelle 2 sind die MHK-Werte in µg/ml des Ciclopiroxolamins in Anwesenheit von 64 µg/ml bzw. 16 µg/ml und 1 µg/ml der Nigericinverbindung angegeben. Die MHK-Werte von Ciclopiroxolamin gegen die angegebenen Pilze sanken dabei auf 0,125 bis 1 µg/ml.

**Tabelle 1:**

| Beispiel Nr. | Formel II A1 | Formel II A2 | Formel III X | Formel IV M1 | Formel IV M2 | Formel IV M3 |
|---|---|---|---|---|---|---|
| 1 | | | | $CH_3$ | $CH_2OH$ | OH |
| 2 | | | | $CH_3$ | $CH_3$ | OH |
| 3 | | | | H | H | OH |
| 4 | 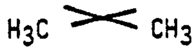 | | | | | |
| 5 | H | $C(O)-CH_3$ | | | | |
| 6 | 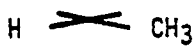 | | | | | |
| 7 | | | $O-Si(C_6H_5)_2(t-Bu)$ | | | |
| 8 | | | Cl | | | |
| 9 | H | C(O)-NH-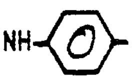-Cl | | | | |
| 10 | | | $O-C_4H_9$ | | | |
| 11 | | | | $NHC_6H_{13}$ | | O |
| 12 | | | | $C_6H_5$ | | O |
| 13 | $CH_3$ | H | | | | |
| 14 | H | C(O)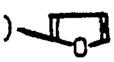 | | | | |
| 15 | H | C(O)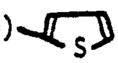 | | | | |
| 16 | H | $C(O)-NH-C_6H_5$ | | | | |
| 17 | H | $C(O)-CH_2-CH_2-COOH$ | | | | |
| 18 | H | $C(O)-C_6H_5$ | | | | |
| 19 | H | $C(O)-CH_2-C_6H_5$ | | | | |
| 20 | H | $C(O)-CH_2-CH_2-O-C_6H_5$ | | | | |

t-Bu = tertiär-Butyl

Tabelle 2

| Nigericin-Verbindung aus Bsp. Nr. | M. canis | | | | C. albicans | | | |
|---|---|---|---|---|---|---|---|---|
| | | MHK [µg/ml] für Batrafen in Anwesenheit von | | | | MHK [µg/ml] für Batrafen in Anwesenheit von | | |
| | MHK µg/ml | 64 µg/ml | 16 µg/ml | 1 µg/ml | MHK µg/ml | 64 µg/ml | 16 µg/ml | 1 µg/ml |
| | | Nigericin/Nigericinderivat | | | | Nigericin/Nigericinderivat | | |
| Ciclopiroxolamin | 2 | | | | 2 | | | |
| Nigericin | 128 | 0,25 | 0,25 | 1 | 128 | 0,5 | 0,5 | 1 |
| 1 | 128 | 1 | 1 | 1 | 128 | 0,5 | 1 | 1 |
| 2 | 128 | 0,5 | 0,5 | 1 | 128 | 0,5 | 1 | 1 |
| 3 | 128 | 0,5 | 0,5 | 1 | 128 | 0,25 | 0,5 | 0,5 |
| 4 | 128 | 0,5 | 0,5 | 1 | 128 | 0,25 | 0,25 | 0,5 |
| 5 | 128 | 1 | 1 | 1 | 128 | 0,25 | 0,25 | 0,5 |
| 6 | 128 | 0,25 | 0,5 | 0,5 | 128 | 0,5 | 0,5 | 0,5 |
| 7 | 128 | 1 | 1 | 1 | 128 | 0,25 | 0,25 | 0,5 |
| 8 | 128 | 0,5 | 0,5 | 1 | 128 | 0,25 | 0,5 | 0,5 |
| 9 | 128 | 0,5 | 0,5 | 0,5 | 128 | 0,5 | 0,5 | 1 |
| 10 | 128 | 0,5 | 0,5 | 0,5 | 128 | 0,25 | 0,5 | 0,5 |
| 11 | 128 | 1 | 1 | 1 | 128 | 0,5 | 0,5 | 0,5 |
| 12 | 128 | 0,5 | 0,5 | 0,5 | 128 | 0,5 | 0,5 | 0,5 |
| 13 | 128 | 0,5 | 0,5 | 1 | 128 | 0,25 | 0,25 | 0,5 |
| 14 | 128 | 0,5 | 0,5 | 1 | 128 | 0,25 | 0,25 | 0,25 |
| 15 | 128 | 0,5 | 0,5 | 0,5 | 128 | 0,25 | 0,5 | 0,5 |
| 16 | 128 | 0,5 | 0,5 | 1 | 128 | 0,5 | 0,5 | 0,5 |
| 17 | 128 | 0,5 | 0,5 | 0,5 | 128 | 0,125 | 0,125 | 0,5 |
| 18 | 128 | 0,5 | 0,5 | 0,5 | 128 | 0,5 | 0,5 | 0,5 |
| 19 | 128 | 0,5 | 0,5 | 1 | 128 | 0,5 | 0,5 | 0,5 |
| 20 | 128 | 0,5 | 1 | 1 | 128 | 0,5 | 0,5 | 0,5 |

**Wirkung der Kombination des Polyethers Nigericin mit Ciclopiroxolamin gegen Herpes simples Virus Typ I und Herpes simples Virus Typ II**

Zellkulturen von Hela- und Vero-Zellen, wurden in Mikrotiterplatten eingesät und mit Herpes simples Virus Typ I bzw. Typ II infiziert. 2 Stunden nach der Infektion wurden die zu prüfenden Verbindungen (Ciclopiroxolamin und Nigericin) den infizierten Zellkulturen in verschiedenen Verdünnungen zugegeben. 48 - 72 Stunden nach der Infektion wurde die minimale Hemmkonzentration anhand des cytopathogenen Effektes mikroskopisch und nach Neutralrotaufnahmen photometrisch bestimmt.

Wie die Figuren 1 und 2 zeigen, wirkt die Kombination von Nigericin und Ciclopiroxolamin sowohl für HSV I als auch für HSV II synergistisch antiviral. Die in den Kurven eingezeichneten Punkte zeigen die Hemmung der Virusvermehrung bei den auf den Achsen angegebenen Mengen an Wirkstoff in µg per ml.

Die verwendeten Nigericinderivate gemäß den Beispielen 1-20 wurden - wie in den deutschen Patentanmeldungen P 3800598.0, P 3811016.4 und P 3820179.8 vorgeschlagen - folgendermaßen hergestellt:

**Allgemeine Verfahrensvorschrift zur Herstellung der in den Beispielen 5, 14, 15, 17, 18, 19 und 20 (s. Tabelle 1) verwendeten Verbindungen:**

725 mg (1 mmol) Nigericin werden in 10 ml Pyridin gelöst und mit 1,5 mmol Anhydrid (welches sich

aus dem Rest $A^2$ ableitet) in Gegenwart von 10 mg Dimethylaminopyridin für 4-24 h umgesetzt. Nach der Zugabe von 40 ml Wasser wird weitere 20 min bei Raumtemperatur gerührt. Die wäßrige Phase wird mit Ethylacetat (3 x 20 ml) extrahiert und die organische Phase wird mit 2 x 10 ml 0,1 N HCl und 2 x 10 ml Wasser gewaschen. Nach dem Trocknen über Natriumsulfat wird im Vakkuum einrotiert. Chromatographie an 250 g Kieselgel mit Chloroform/Methanol von 30 : 1 auf 9 : 1 ergibt die Ester.

**Allgemeine Verfahrensvorschrift zur Herstellung der in den Beispielen 16 und 9 (s. Tabelle 1) verwendeten Verbindungen:**

725 mg (1 mmol) Nigericin werden in 20 ml Chloroform gelöst und mit 1,5 mmol Isocyanat (welches sich aus dem Rest $A^2$ ableitet) für 24 h bei Raumtemperatur gerührt. Nach der Zugabe von Wasser (50 ml) wird mit 2 x 20 ml Chloroform extrahiert. Nach dem Trocknen über Natriumsulfat wird bis zu einem Sirup einrotiert. Chromatographie an 250 g Kieselgel mit Chloroform/Methanol von 30 : 1 auf 9 : 1 ergibt die Carbaminsäure-Derivate.

**Allgemeine Verfahrensvorschrift zur Herstellung der in Beispiel 13 (s. Tabelle 1) verwendeten Verbindung:**

725 mg ( 1mmol) Nigericin werden in 10 ml Alkohol (welcher sich aus dem Rest $A^1$ ableitet) gelöst und mit 100 mg Lithiumbromid für 2 h unter Rückfluß erhitzt. Anschließend wird bis zur Trockne eingedampft. Der Rückstand wird in 20 ml Ethylacetat aufgenommen und mit Wasser (2 x 20 ml) extrahiert. Nach dem Einrotierten wird das Ketal als fester Rückstand erhalten.

**Allgemeine Verfahrensvorschrift zur Herstellung der in den Beispielen 4 und 6 (s. Tabelle 1) verwendeten Verbindungen:**

725 mg (1 mmol) Nigericin werden in 10 ml Carbonylverbindung (Aceton bzw. Acetaldehyd) gelöst und mit 200 mg Zinkchlorid unter Rückfluß erhitzt. Anschließend wird bis zur Trockne eingedampft. Der Rückstand wird in 20 ml Ethylacetat aufgenommen und mit 2 x 20 ml Wasser gewaschen. nach den Einrotieren wird das Ketal als fester Rückstand erhalten.

**Allgemeine Verfahrensvorschrift zur Herstellung der Verbindung der Formel V und der in Beispiel 12 (s. Tabelle 1) verwendeten Verbindung:**

725 mg (1 mmol) Nigericin [zur Herstellung der Verbindung der Formel V] oder Nigericinderivat der Formel IV, mit $M^3$ = OH, $M^2$ = $CH_2OH$ und $M^1$ = $C_6H_5$ (erhältlich durch Umsetzung von $C_6H_5MgCl$ mit Nigericin, wie für Beispiel 1 beschrieben) [Zur Herstellung des Nigericin-Derivats gemäß Beispiel 12] wird in 30 ml Tetrahydrofuran und 30 ml Wasser suspendiert und mit 4 g $NaIO_4$ für 30 h bei Zimmertemperatur gerührt. Nach dem Abziehen des Tetrahydrofurans im Vakuum wird die wäßrige Phase mit 3 x 50 ml Ethylacetat extrahiert. Nach dem Trocknen über Natriumsulfat wird bis zur einem Sirup eingeengt. Chromatographie an 300 g Kieselgel mit einem Gradienten Chloroform/Methanol 50 : 1 auf 9 : 1 (3 Liter) lieferte die entsprechenden Derivate.

**Allgemeine Verfahrensvorschrift zur Herstellung der in Beispiel 1 und 2 (s. Tabelle 1) verwendeten Verbindungen:**

1 mmol Nigericin [zur Herstellung des Nigericinderivats gemäß Beispiel 1] oder Nigericinderivat der Formel V (erhältlich wie oben beschrieben) [zur Herstellung des Nigericinderivats gemäß Beispiel 2] wird in 50 ml Tetrahydrofuran gelöst und bei 0 °C 30 h mit 8 mmol Grignardreagenz gerührt. Nach der Hydrolyse mit 50 ml Wasser wird das Tetrahydrofuran im Vakuum abdestilliert. Die wäßrige Phase wird mit 3 x 50 ml Ethylacetat extrahiert, die organische Phase wird mit 0,1 N Salzsäure gewaschen und anschließend über Natriumsulfat getrocknet. Nach dem Abziehen des Lösungsmittels im Vakuum wird der Rückstand an 300 g Kieselgel chromatographiert. Elution mit $CHCl_3/CH_3OH$ von 30 : 1 auf 1 : 1 liefert die entsprechenden

Derivate.

**Allgemeine Verfahrensvorschrift zur Herstellung der in Beispiel 3 (s. Tabelle 1) verwendeten Verbindung:**

1 mmol Nigericinderivat der Formel V wird in 50 ml Tetrahydrofuran mit 3 mmol Lithiumaluminiumhydrid umgesetzt. Nach vorsichtiger Zugabe von 50 ml Wasser und 10 ml 5 N HCl wird das THF im Vakuum abdestilliert. Extraktion mit 3 x 50 ml Ethylacetat, Waschen der organischen Phase mit 10 ml 2N HCl, Trocknen über Natriumsulfat und Abdestillieren des Lösungsmittels ergibt einen Rückstand, der an 300 g Kieselgel chromatographiert wird. Elution mit einem Gradienten Chloroform/Methanol von 30 : 1 auf 9 : 1 ergibt die entsprechenden Alkohole.

**Allgemeine Verfahrensvorschrift zur Herstellung der in Beispiel 11 (s. Tabelle 1) verwendeten Verbindung:**

1 mmol Nigericinderivat der Formel V wird in 40 ml Tetrahydrofuran mit 5 mmol des primären Amins 20 h bei Zimmertemperatur gerührt. Nach dem Abdestillieren des Lösungsmittels im Vakuum wird an 300 g Kieselgel chromatographiert. Elution mit Chloroform/Methanol Gradient von 50:1 auf 9:1 ergibt die Amide.

**Allgemeine Verfahrensvorschrift zur Herstellung von Nigericinmesylat (Formel III, X = $OSO_2CH_3$):**

725 mg (1 mmol) Nigericin werden mit 1,5 mmol $ClSO_2CH_3$ für 0,5 h in Pyridin (10 ml) gerührt. Nach der Zugabe von Wasser wird weitere 20 min bei Raumtemperatur gerührt. Die wäßrige Phase wird mit Ethylacetat (3 x 20 ml) extrahiert und die organische Phase wird mit 2 x 10 ml 0,1 N HCl und 2 x 10 ml Wasser gewaschen. Nach dem Trocknen über Natriumsulfat wird im Vakuum einrotiert. Chromatographie an 250 g Kieselgel mit Chloroform/Methanol von 30 : 1 auf 9 : 1 ergibt das Nigericinmesylat.

**Allgemeine Verfahrensvorschrift zur Herstellung der in Beispiel 8 (s. Tabelle 1) verwendeten Verbindung:**

0,1 mmol Nigericinmesylat (Formel III, X = $O\text{-}SO_2\text{-}CH_3$) werden in 30 ml Toluol gelöst und mit 5 mmol Tetrabutylammoniumchlorid für 3 - 40 h auf 60 °C erhitzt. Die organische Phase wird 2 mal mit 20 ml Wasser gewaschen und anschließend im Vakuum einrotiert. Chromatographie an Kieselgel mit einem Gradienten von $CHCl_3$/MeOH von 40 : 1 auf 9 : 1 ergibt die reine Verbindung.

**Allgemeine Verfahrensvorschrift zur Herstellung der in Beispiel 10 (s. Tabelle 1) verwendeten Verbindung:**

1 mmol Nigericinmesylat werden in 30 ml Butanol gelöst und 1 mmol NaH 6 - 10 h auf 60 °C erhitzt. Nach dem Neutralisieren mti $NH_4Cl$ wird bis zu einem Sirup eingedampft und wie zuvor für die Herstellung der Verbindung gemäß Beispiel 8 beschrieben, chromatographiert.

**Allgemeine Verfahrensvorschrift zur Herstellung der in Beispiel 7 (s. Tabelle 1) verwendeten Verbindung:**

1 mmol Nigericin werden in 30 ml Pyridin gelöst und mit 1,3 mmol t-Butyldiphenylsilylchlorid für 20 h bei Raumtemperatur gerührt. Nach der Hydrolyse mit Wasser (100 ml) wird mit Ethylacetat (3 mal 30 ml) extrahiert. Die organische Phase wird mit 50 ml Wasser und 50 ml 0,1 N HCl gewaschen. Nach dem Trocknen wird die Lösung einrotiert und wie zuvor für die Herstellung der Verbindung gemäß Beispiel 8 beschrieben, chromatographiert.

**Allgemeine Verfahrensvorschrift zur Herstellung von Ciclopiroxolamin (1-Hydroxy-4-methyl-6-cyclohexyl-2(1H)-pyridon):**

11,2 g des durch Kondensation von Hexahydrobenzoylchlorid mit $\beta,\beta$-Dimethylacrylsäure-methylester gewonnen Gemisches aus 5-Oxo-3-methyl-5-cyclohexyl-penten-(2)-säure-(1)-methylester und 5-Oxo-3-methyl-5-cyclohexyl-penten-(3)-säure-(1)-methylester werden mit einer Lösung von 4,6 g Natriumacetat und 4 g Hydroxylamin-hydrochlorid in einem Gemisch aus 8 ml Wasser und 15 ml Methanol 20 Stunden bei 25 °C geschüttelt. Anschließend wird unter Kühlung eine Lösung von 4 g Natriumhydroxyd in 8 ml Wasser zugegeben, noch 1 Stunde bei Raumtemperatur geschüttelt, mit Benzol extrahiert und die wäßrige Phase auf einen pH-Wert von 6 angesäuert. Man erhält 3,5 g 1-Hydroxy-4-methyl-6-cyclohexyl-2-(1H)-pyridon vom Schmelzpunkt 144 °C.

## Ansprüche

1) Arzneimittel mit synergistischer antimykotischer und antiviraler Wirksamkeit, dadurch gekennzeichnet, daß es
$\alpha$) mindestens eine Verbindung, ausgewählt aus:
Ciclopirox und Ciclopiroxderivaten der Formel I

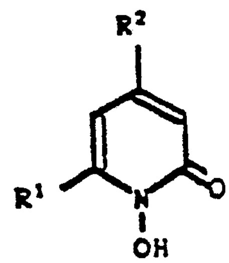

(I)

worin
$R^1$ Wasserstoff, $C_1$-$C_{10}$-Alkyl oder $C_4$-$C_8$-Cycloalkyl und
$R^2$ Wasserstoff oder $C_1$-$C_4$-Alkyl bedeuten
und den physiologisch verträglichen Salzen dieser Verbindungen
und
$\beta$) mindestens eine Verbindung, ausgewählt aus:
Nigericin und den Nigericinderivaten der Formeln II, III, IV und V

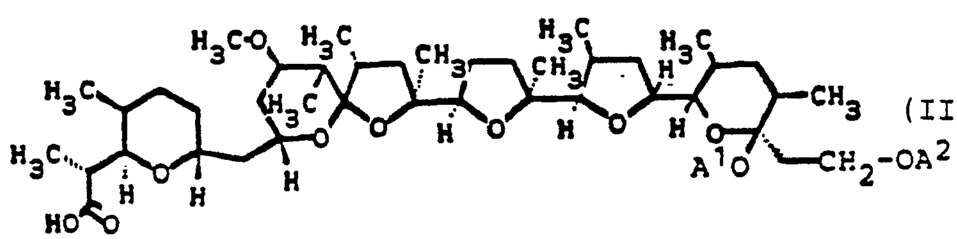

(II)

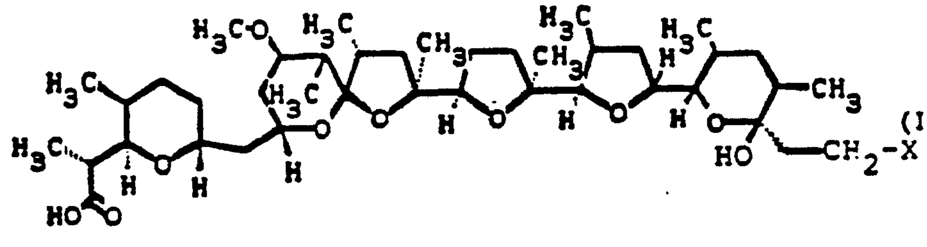

(III)

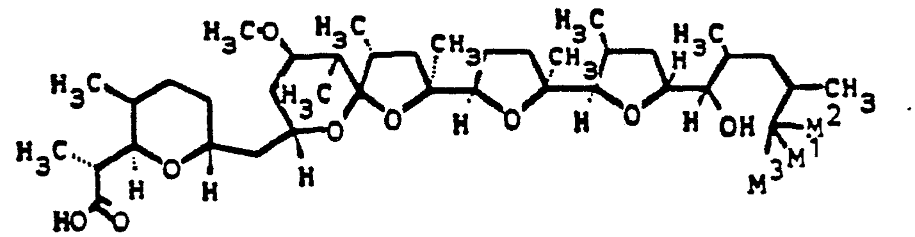

(IV)-

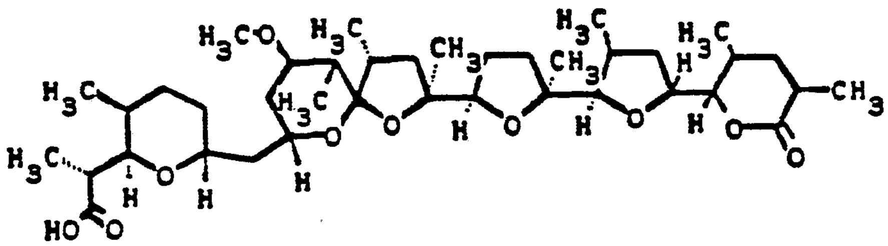

(V)

worin

$A^1$ Wasserstoff oder $C_1$-$C_8$-Alkyl bedeutet und

$A^2$ Wasserstoff bedeutet oder einen Rest der Formel

$-\overset{\overset{O}{\|}}{C}-A^3$, $-\overset{\overset{O}{\|}}{C}-NH-A^3$ oder $-\overset{\overset{O}{\|}}{C}-O-A^3$ darstellt, in der

$A^3$ $C_1$-$C_{15}$-Alkyl bedeutet, welches gegebenenfalls halogen-, nitro-, cyano-, carboxyl-, $C_1$-$C_4$-alkoxy-, phenyloxy oder phenylsubstituiert ist, wobei die genannten Phenylreste ihrereseits mit Halogen, Nitro, Cyano, $C_1$-$C_4$-Alkyl oder $C_1$-$C_4$-Alkoxy substituiert sein können, oder in der

$A^3$ Phenyl, Furyl oder Thienyl bedeutet, wobei diese Phenyl,- Furyl- oder Thienyl-Reste mit Halogen, Nitro, Cyano, $C_1$-$C_4$-Alkyl oder $C_1$-$C_4$-Alkoxy substituiert sein können

oder worin

$A^2$ einen Sulfonsäureester der Formel $-SO_2A^4$ darstellt, worin

$A^4$ Hydroxy, $C_1$-$C_6$-Alkyl, Phenyl oder p-Tolyl bedeutet

oder worin

$A^1$ und $A^2$ zusammen einen Rest der Formel VI darstellen

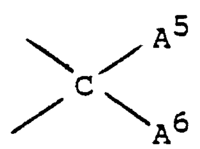

(VI)

in der

$A^5$ und $A^6$ gleich oder verschieden sind und Wasserstoff oder $C_1$-$C_{10}$-Alkyl bedeuten oder wobei

$A^5$ und $A^6$ eine Alkylenkette darstellen, die zusammen mit dem sie tragenden C-Atom einen 5-, 6- oder 7-gliedrigen Ring bildet,

X F, Cl, Br, J, SCN, CN, $NO_2$ oder $N_3$ bedeutet oder einen Substituenten der Formel VII darstellt

$Y - L^1$ (VII)

worin

$L^1$ Wasserstoff, $C_1$-$C_{10}$-Alkyl, $C_2$-$C_{10}$-Alkenyl, $C_3$-$C_8$-Cycloalkyl, Trimethylsilyl, Triethylsilyl, Dimethylphenylsilyl, Dimethylcyclohexylsilyl, Dimethyltertiärbutylsilyl, Diphenyltertiärbutylsilyl, Pyrrolyl, Benzpyrrolyl, Imidazolyl, Benzimidazolyl, Triazolyl, Tetrazolyl oder Phenyl bedeutet, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN mono- oder disubstituiert sind und worin

Y O oder S bedeutet, wobei für $OL^1$ die Bedeutungen Wasserstoff, Pyrrol, Benzpyrrol, Imidazol, Benzimidazol, Triazol und Tetrazol für $L^1$ ausgenommen sind und für

$SL^1$ die Bedeutungen Trimethylsilyl, Triethylsilyl, Dimethylphenylsilyl, Dimethylcyclohexylsilyl, Diphenyltertiärbutylsilyl und Dimethyltertiärbutylsilyl für $L^1$ ausgenommen sind oder worin
X einen Rest der Formel VIII

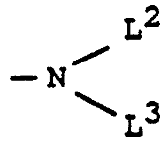

(VIII)

darstellt,
worin
$L^2$ und $L^3$ gleich oder verschieden sind und Wasserstoff, $C_1$-$C_{18}$-Alkyl, $C_2$-$C_{18}$-Alkenyl, $C_3$-$C_8$-Cycloalkyl oder Phenyl bedeuten, wobei die genannten Alkyl-, Alkenyl- und Cycloalkyl-Reste ihrerseits mit Phenyl, Naphthyl oder Thienyl monosubstituiert und die genannten Phenyl- und Alkylreste darüber hinaus noch mit $COOL^4$ monosubstituiert sein können, wobei $L^4$ Wasserstoff oder $C_1$-$C_6$-Alkyl bedeutet oder worin
$L^2$ und $L^3$ zusammen mit dem N-Atom, an dem sie gebunden sind, einen 5- oder 6-gliedrigen N-haltigen Alkylring bilden, wobei eine $CH_2$-Einheit des Rings, welche nicht direkt benachbart zum N-Atom steht, ihrerseits durch NH, O oder S ersetzt sein kann,
$M^1$ Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert ist durch ($C_1$-$C_{10}$)-Alkyl, ($C_1$-$C_{10}$)-Alkoxy oder Phenoxy,
$M^2$ Wasserstoff, ($C_1$-$C_{18}$)-Alkyl (geradkettig oder verzweigt, gesättigt oder ungesättigt), ($C_3$-$C_8$)-Cycloalkyl, Phenyl (unsubstituiert oder substituiert durch ($C_1$-$C_{10}$)-Alkyl, ($C_1$-$C_{10}$)-Alkoxy oder Phenoxy), $CH_2OH$,
$M^3$ OH,
bedeuten,
oder in der
$M^2$ und $M^3$ gemeinsam Sauerstoff und
$M^1$ Wasserstoff, ($C_1$-$C_{18}$)-Alkyl (geradkettig oder verzweigt, gesättigt oder ungesättigt), ($C_3$-$C_8$)-Cycloalkyl, Phenyl (unsubstituiert oder substituiert durch ($C_1$-$C_{10}$)-Alkyl, ($C_1$-$C_{10}$)-Alkoxy oder Phenoxy) oder
$NM^4$ $M^5$ bedeuten, worin
$M^4$ und $M^5$ gleich oder verschieden sind und folgende Bedeutungen haben:
i) Wasserstoff,
ii) ($C_1$-$C_{18}$)-Alkyl (gesättigt oder ungesättigt, geradkettig oder verzweigt), wobei die Alkylgruppen unsubstituiert sind oder substituiert mit F, Cl, Br, Phenyl, Naphthyl, Thienyl, $COOM^6$ oder $CON(M^6)_2$ mit $M^6$ gleich Wasserstoff oder ($C_1$-$C_6$)-Alkyl,
iii) ($C_3$-$C_8$)-Cycloalkyl,
iv) Arylgruppen mit insgesamt 6-20 C-Atomen, worin Aryl, Phenyl, Naphthyl, Thienyl bedeutet, welche Systeme entweder unsubstituiert sind oder substituiert mit geradkettigem oder verzweigtem Alkyl mit bis zu 14 C-Atomen, oder F, Cl, Br, J, Nitro, Cyano, Alkoxy, Phenoxy,
und den physiologisch verträglichen Salzen der Verbindungen der Formeln II, III, IV und V, enthält.

2) Arzneimittel nach Anspruch 1, dadurch gekennzeichnet, daß mindestens eine der nachfolgenden Bedingungen erfüllt ist:
$R^1$ bedeutet Wasserstoff, $C_5$-$C_8$-Alkyl oder $C_5$-$C_6$-Cycloalkyl,
$R^2$ bedeutet Wasserstoff oder $C_1$-$C_3$-Alkyl;
$A^1$ bedeutet Wasserstoff oder $C_1$-$C_4$-Alkyl,
$A^3$ bedeutet $C_1$-$C_{12}$-Alkyl, welches gegebenenfalls carboxy-, phenyloxy- oder phenylsubstituiert ist, wobei die genannten Phenylreste ihrerseits mit Halogen substituiert sein können, oder
$A^3$ bedeutet Phenyl, Furyl oder Thienyl, wobei diese Phenyl-, Furyl- oder Thienylreste mit Halogen substituiert sein können,
$A^4$ bedeutet Hydroxy oder $C_1$-$C_4$-Alkyl,
$A^5$ und $A^6$ sind gleich oder verschieden und bedeuten Wasserstoff oder $C_1$-$C_4$-Alkyl;
$L^1$ bedeutet Wasserstoff, $C_1$-$C_8$-Alkyl, $C_3$-$C_8$-Alkenyl, $C_3$-$C_8$-Cycloalkyl, Trimethylsilyl, Triethylsilyl, Dimethyltertiärbutylsilyl, Dimethylphenylsilyl, Dimethylcyclohexylsilyl, Dimethyltertiärbutylsilyl, Pyrrolyl, Benzpyrrolyl, Tetrazolyl oder Phenyl, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN monosubstituiert sind;

$L^2$ und $L^3$ sind gleich oder verschieden und bedeuten Wasserstoff, $C_1$-$C_8$-Alkyl, $C_5$-$C_6$-Cycloalkyl oder Phenyl, wobei die genannten Alkyl- und Cycloalkylreste ihrerseits mit Phenyl monosubstituiert und die Phenyl- und Alkylrest darüber hinaus noch mit $COOL^4$ monosubstituiert sein können, wobei $L^4$ Wasserstoff oder $C_1$-$C_4$-Alkyl bedeutet;
$M^1$ bedeutet Wasserstoff, $C_1$-$C_7$-Alkyl, Phenyl oder $NM^4 M^5$, wobei $M^4$ und $M^5$ $C_1$-$C_6$-Alkyl, Phenyl oder Wasserstoff bedeuten;
$M^2$ bedeutet $C_1$-$C_6$-Alkyl, Wasserstoff, Phenyl oder $CH_2OH$;
$M^3$ bedeutet OH oder
$M^2$ und $M^3$ bedeuten gemeinsam Sauerstoff.

3) Arzneimittel nach Anspruch 1 und/oder 2, dadurch gekennzeichnet, daß mindestens eine der nachfolgenden Bedingungen erfüllt ist:
$R^1$ bedeutet verzweigtes oder unverzweigtes $C_8$-Alkyl oder Cyclohexyl
$R^2$ bedeutet Wasserstoff oder Methyl;
$A^1$ bedeutet Wasserstoff, Methyl oder Ethyl,
$A^3$ bedeutet $C_1$-$C_{11}$-Alkyl, welches gegebenenfalls carboxy-, phenyloxy- oder phenylsubstituiert ist, oder
$A^3$ bedeutet Phenyl, Furyl oder Thienyl, wobei der Phenylrest mit Chlor substituiert sein kann,
$A^4$ bedeutet Hydroxy oder Methyl,
$A^5$ und $A^6$ sind gleich oder verschieden und bedeuten Wasserstoff oder Methyl;
X bedeutet F, Cl, Br, SCN, CN, $NO_2$ oder $N_3$ oder einen Substituenten der Formel VII, worin
$L^1$ Wasserstoff, $C_1$-$C_6$-Alkyl, $C_5$-$C_6$-Cycloalkyl, Trimethylsilyl, Triethylsilyl, Diphenyltertiärbutylsilyl, Dimethylphenylsilyl, Dimethylcyclohexylsilyl, Dimethyltertiärbutylsilyl, Pyrrolyl, Benzpyrrolyl, Tetrazolyl, Methyltetrazolyl oder Phenyl bedeutet,
$L^2$ und $L^3$ sind gleich oder verschieden und bedeuten Wasserstoff, $C_1$-$C_4$-Alkyl oder Phenyl, wobei die Alkylreste ihrerseits mit Phenyl monosubstituiert sein können,
$L^2$ und $L^3$ bilden zusammen mit dem N-Atom, an dem sie gebunden sind, einen Morpholin-, Thiomorpholin- oder Piperazinring;
$M^1$ bedeutet Wasserstoff oder $C_1$-$C_3$-Alkyl
$M^2$ bedeutet Wasserstoff, $C_1$-$C_3$-Alkyl oder $CH_2OH$
$M^1$ und $M^2$ sind gleich und bedeuten Wasserstoff oder $C_1$-$C_3$-Alkyl
$M^3$ bedeutet OH
$M^2$ und $M^3$ bedeutet gemeinsam Sauerstoff, wobei gleichzeitig $M^1$ $C_1$-$C_6$-Alkyl oder $NM^4M^5$ bedeutet, mit $M^4$ = Wasserstoff und $M^5$ = $C_1$-$C_6$-Alkyl.

4) Verfahren zur Herstellung eines synergistisch antimykotisch und antiviral wirkenden Arzneimittels, dadurch gekennzeichnet, daß man pharmazeutisch wirksame Mengen mindestens einer Verbindung ausgewählt aus den Verbindungen der Formel I und deren physiologisch verträglichen Salzen nach Anspruch 1 mit pharmazeutisch wirksamen Mengen mindestens einer Verbindung ausgewählt aus den Verbindungen der Formeln II, III, IV und V und deren physiologisch verträglichen Salze nach Anspruch 1 und gegebenenfalls mit pharmazeutischen Hilfs- und/oder Trägerstoffen und/oder Lösungsvermittlern und/oder Lösungsmitteln vermischt und in eine geeignete Darreichungsform überführt.

5) Verwendung von mindestens einer Verbindung ausgewählt aus Verbindungen der Formel I und deren physiologisch verträglichen Salze in Kombination mit mindestens einer Verbindung ausgewählt aus den Verbindungen der Formeln II, III, IV, V und deren physiologisch verträgliche Salze, als synergistisch antimykotisch und antiviral wirkende Arzneimittel.

Fig. 1

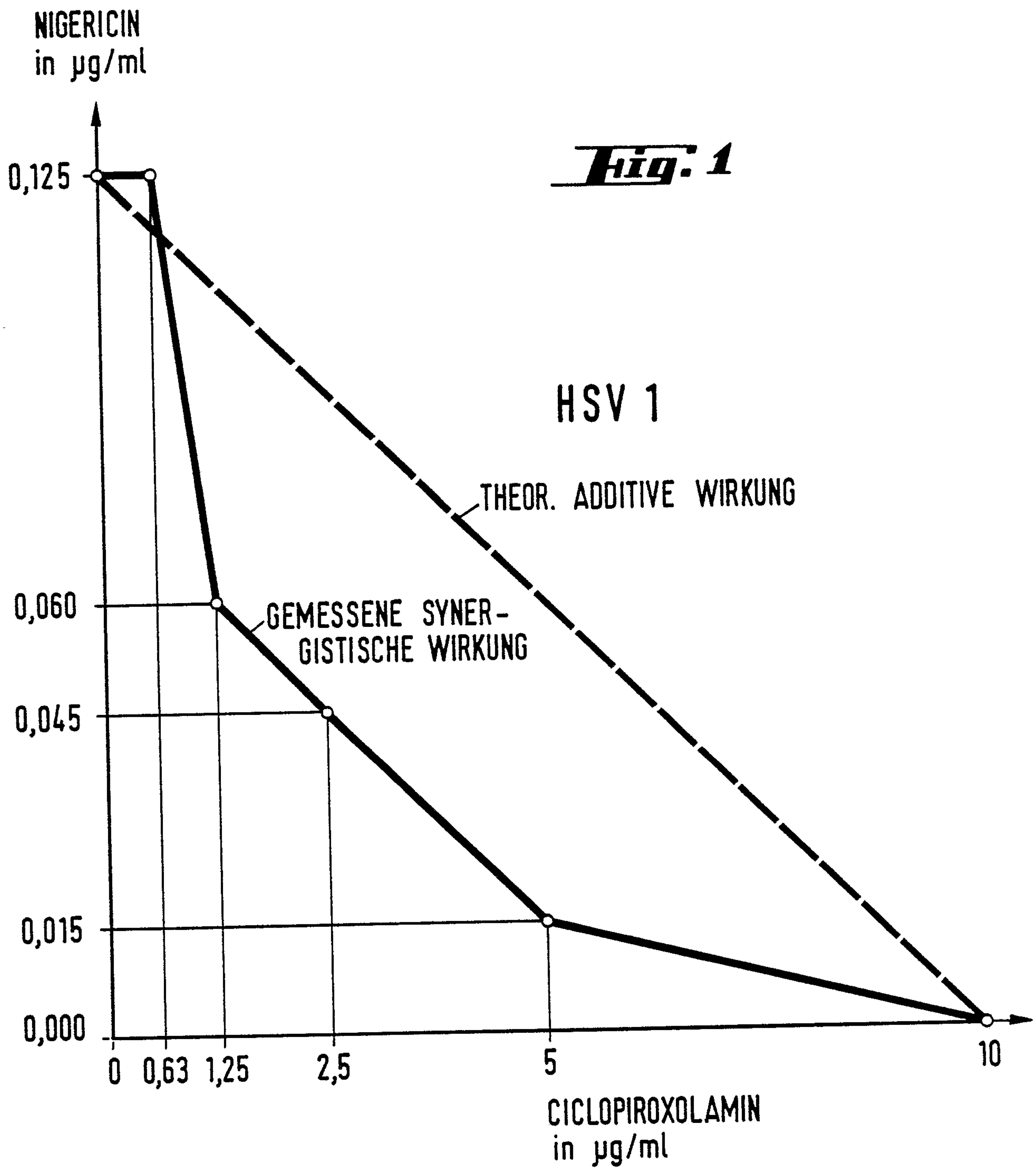
NIGERICIN
in µg/ml
0,125
0,060
0,045
0,015
0,000
HSV 1
THEOR. ADDITIVE WIRKUNG
GEMESSENE SYNER-
GISTISCHE WIRKUNG
0
0,63
1,25
2,5
5
10
CICLOPIROXOLAMIN
in µg/ml

Fig. 2

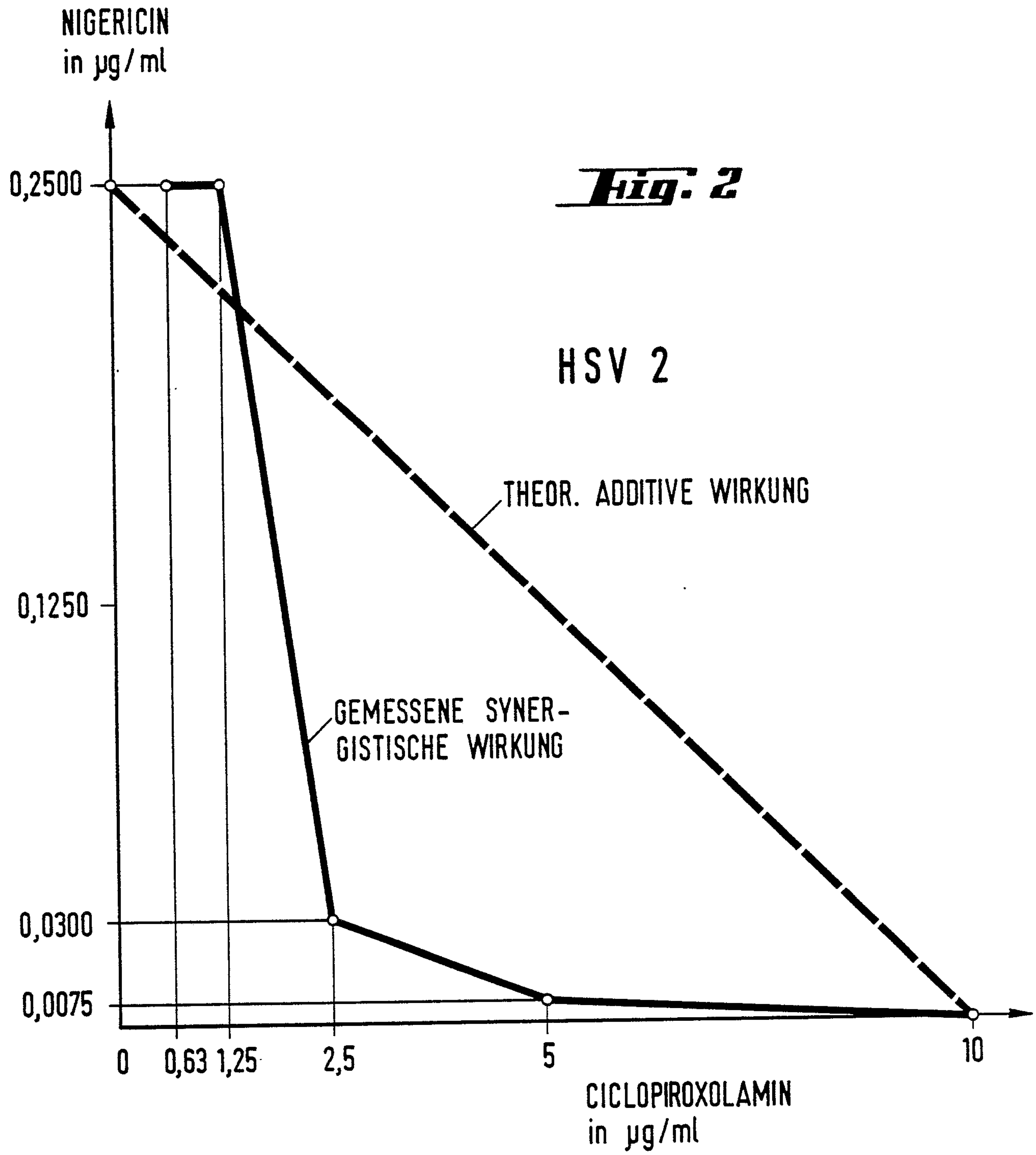
NIGERICIN
in µg/ml
0,2500
0,1250
0,0300
0,0075
HSV 2
THEOR. ADDITIVE WIRKUNG
GEMESSENE SYNER-
GISTISCHE WIRKUNG
0
0,63
1,25
2,5
5
10
CICLOPIROXOLAMIN
in µg/ml

Europäisches Patentamt

# EUROPÄISCHER RECHERCHENBERICHT

Nummer der Anmeldung

EP 89 11 6418

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| E | FR-A-2 618 072 (S.A. L'OREAL)<br>* Seiten 22-25; Ansprüche 1-20 *<br>--- | 1-5 | A 61 K 31/71 //<br>(A 61 K 31/71<br>A 61 K 31:44 ) |
| D,A | ARZNEIM.-FORSCH./DRUG RES., Band 31 (II), Nr. 8a, 1981, Seiten 1360-1365; W. ADAM et al.: "Klinische Ergebnisse mit dem Antimykotikum Ciclopiroxolamin"<br>* Seite 1364, Punkt 3.4 Gesamtergebnis, Punkt 4. Diskussion *<br>----- | 1-5 | |

| RECHERCHIERTE SACHGEBIETE (Int. Cl.5) |
|---|
| A 61 K |

Der vorliegende Recherchenbericht wurde für alle Patentansprüche erstellt

| Recherchenort | Abschlußdatum der Recherche | Prüfer |
|---|---|---|
| DEN HAAG | 27-11-1989 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)